# EUROPEAN PATENT APPLICATION

(11) **EP 2 468 306 A1**
(43) Date of publication of application: **27.06.2012**
(21) Application number: 10015886.4
(22) Date of filing: 21.12.2010
(51) Int. Cl.: A61L 15/28, A61L 15/26, A61L 15/46

(54) **Absorbent article comprising cyclodextrin complex**

(71) Applicant: THE PROCTER & GAMBLE COMPANY, Cincinnati, OH 45202 (US)
(72) Inventor: Caputi, Marinangela, 65126 Pescara (IT); Pourcel, Magali, 65125 Pescara (IT)
(74) Representative: Kremer, Véronique Marie Joséphine

(57) **Abstract**

An absorbent article comprises a topsheet, a backsheet, an absorbent core disposed between the topsheet and backsheet, and a cyclodextrin complex that is disposed in a layer of the absorbent article that is closer to the body-facing surface, of the absorbent article than the absorbent core. The cyclodextrin complex comprises cyclodextrin and at least three components complexed with the cyclodextrin.

## Description

### FIELD OF THE INVENTION

The present invention relates to an absorbent article comprising a cyclodextrin complex comprising cyclodextrin and at least three components complexed with the cyclodextrin.

### BACKGROUND OF THE INVENTION

Absorbent articles for personal hygiene are known in the art. Typical examples include sanitary napkins, pantiliners, tampons, inter labial articles, adult incontinence articles, and baby diapers. Such articles are commonly used to absorb and retain bodily fluids and other exudates excreted by the human body, such as urine and menses. Typically, such exudates are perceived as malodorous and offensive. Therefore, methods and materials for controlling and reducing malodors in absorbent articles have been developed. Fragrance materials have been widely used for this purpose in absorbent articles, as well as ingredients such as silica or zeolites which are able to entrap some of the malodour generating molecules. The use of fragrance materials, however, tends to provide an overwhelming perfume scent to the product before use that may not be acceptable to some consumers. There thus still remains a desire to provide an improved malodor control composition for incorporation into an absorbent article product.

There remains a need to develop a malodor control technology that does not impart a perceptible odor to the absorbent article before use and that efficiently provides malodor control benefits throughout the period of time that the absorbent article is typically worn by a consumer.

### SUMMARY OF THE INVENTION

The present invention relates to an absorbent article comprising a topsheet, a backsheet, an absorbent core disposed between the topsheet and backsheet, and a cyclodextrin complex that is disposed in a layer of the absorbent article that is closer to the body-facing surface of the absorbent article than the absorbent core. The cyclodextrin complex comprises cyclodextrin and at least three components complexed with the cyclodextrin.

The absorbent article of the present invention exhibits no, or very little, scent prior to use. Upon use, the bodily fluid contacts the cyclodextrin complex and provides an effective release of the components of the cyclodextrin complex in order to reduce the malodor associated with the bodily fluid. The present invention can provide sustained odor control for the period of time the absorbent article is typically worn by a consumer, which is typically about 4 hours during the daytime and typically about 8 hours overnight.

The present invention further relates to a method of reducing the malodor associated with bodily fluids, such as urine, menses, and/or feces, comprising the step of contacting the bodily fluid with an absorbent article of the present invention.

### BRIEF DESCRIPTION OF THE DRAWINGS

FIG. 1 is a top view of an absorbent article of the present invention.
FIG. 2 is a cross-sectional view of the absorbent article of FIG. 1.
FIG. 3 is a front view of a glass vial containing an absorbent article sample for the Headspace Test Method.
FIG. 4 is a graph showing the Total Headspace Area of components originally complexed with cyclodextrin as a function of time after insult of Artificial Menstrual Fluid.

### DETAILED DESCRIPTION OF THE INVENTION

"Absorbent article" refers to devices that absorb and contain body exudates, such as urine, menses, and feces. The term "disposable" is used herein to describe absorbent articles which are not intended to be laundered or otherwise restored or reused as an absorbent article after a single use. Examples of absorbent articles include diapers, toddler training pants, adult incontinence garments, and feminine hygiene garments such as sanitary napkins, pantiliners, interlabial devices, hemorrhoid pads, and the like.

Absorbent articles and components thereof, including the topsheet, backsheet, absorbent core, and any individual layers of these components, have a body-facing surface and a garment-facing surface. As used herein, "body-facing surface" means that surface of the article or component which is intended to be worn toward or adjacent to the body of the wearer, while the "garment-facing surface" is on the opposite side and is intended to be worn toward or placed adjacent to the wearer's undergarments when the disposable absorbent article is worn.

### ABSORBENT ARTICLE

In general, the absorbent articles of the present invention typically comprise a topsheet, a backsheet, and an absorbent core disposed between the topsheet and backsheet.

The topsheet of the absorbent article is preferably compliant, soft feeling, and nonirritating to the wearers skin and hair. Further, the topsheet is liquid pervious, permitting liquids (e.g., menses and/or urine) to readily penetrate through its thickness. A suitable topsheet may be manufactured from a wide range of materials such as woven and nonwoven materials (e.g., a nonwoven web of fibers); polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, carded, wet-laid, melt-blown, hydroentangled, combinations of the above, or the like.

The backsheet is impervious to liquids (e.g., menses and/or urine) and is preferably manufactured from a thin plastic film, although other flexible liquid impervious materials may also be used. As used herein, the term "flexible" refers to materials which are compliant and will readily conform to the general shape and contours of the human body. The backsheet prevents the exudates absorbed and contained in the absorbent core from wetting articles which contact the absorbent article such as bedsheets, pants, pajamas and undergarments. The backsheet can also be vapor permeable ("breathable"), while remaining fluid impermeable. The backsheet may comprise a woven or nonwoven material, polymeric films such as thermoplastic films of polyethylene or polypropylene, or composite materials such as a film-coated nonwoven material.

The backsheet and the topsheet can positioned adjacent the garment surface and the body surface, respectively, of the absorbent core. The absorbent core can be joined with the topsheet, the backsheet, or both in any manner as is known by attachment means such as those well known in the art. Embodiments of the present invention are envisioned wherein portions of the entire absorbent core are unattached to either the topsheet, the backsheet, or both.

The absorbent core can be formed from any of the materials well known to those of ordinary skill in the art. Examples of such materials include multiple plies of creped cellulose wadding, fluffed cellulose fibers, wood pulp fibers also known as airfelt, textile fibers, a blend of fibers, a mass or batt of fibers, airlaid webs of fibers, a web of polymeric fibers, and a blend of polymeric fibers. Other suitable absorbent core materials include absorbent foams such as polyurethane foams or high internal phase emulsion ("HIPE") foams. Suitable HIPE foams are disclosed in US 5,550,167, US 5,387,207, US 5,352,711, and US 5,331,015.

For some absorbent articles, the absorbent core can be relatively thin, less than about 5 mm in thickness, or less than about 3 mm, or less than about 1 mm in thickness. Thickness can be determined by measuring the thickness at the midpoint along the longitudinal centerline of the pad by any means known in the art for doing while under a uniform pressure of 1.72 kPa.

The absorbent core can comprise superabsorbent materials such as absorbent gelling materials (AGM), including AGM fibers, as is known in the art. The absorbent core can therefore constitute a layer comprising superabsorbent material.

The absorbent article can comprise other additional components, for example between the topsheet and absorbent core, such as a secondary topsheet or acquisition layer. The secondary topsheet or acquisition layer can comprise a tissue layer or a nonwoven, such as carded resin-bonded nonwovens, embossed carded resin-bonded nonwovens, high-loft carded resin-bonded nonwovens, carded through-air-bonded nonwovens, carded thermo-bonded nonwovens, spunbonded nonwovens, and the like. A variety of fibers can be used in the secondary topsheet or acquisition layer, including natural fibers, e.g. wood pulp, cotton, wool, and the like, as well as biodegradeable fibers, such as polylactic acid fibers, and synthetic fibers such as polyolefins (e.g., polyethylene and polypropylene), polyesters, polyamides, synthetic cellulosics (e.g., RAYON®, Lyocell), cellulose acetate, bicomponent fibers, and blends thereof. The basis weight of the secondary topsheet or acquisition layer can vary depending upon the desired application.

The absorbent article can comprise further components such as side cuffs, typically found in diapers, or side wings or side flaps, typically found in sanitary napkins.

The absorbent articles herein are preferably disposable after a single use.

The cyclodextrin complex of the present invention can be disposed in various locations in the absorbent article. The cyclodextrin complex can be disposed on the garment-facing side or the body-facing side of the topsheet or absorbent core, or the body-facing side of the backsheet. Preferably, the cyclodextrin complex is disposed on the absorbent core, and preferably on the body-facing side of the absorbent core. The malodor control composition can also be disposed on other components, when present in the absorbent article, such as the garment-facing side or body-facing side of a secondary topsheet or acquisition layer.

The cyclodextrin complex of the present invention is disposed in the absorbent article in a layer that is closer to the body-facing surface of the absorbent article than the absorbent core or a layer comprising superabsorbent material (e.g. absorbent gelling material ("AGM")). In order for the cyclodextrin complex to effectively release the components of the cyclodextrin complex, the complex needs to come in contact with moisture. A problem exists when incorporating a cyclodextrin complex in an absorbent article, because other components, such as the absorbent core and/or superabsorbent material, of the absorbent article have a strong affinity for bodily fluids, including the moisture contained therein. When an absorbent article is insulted with bodily fluid, such as menses or urine, the cyclodextrin complex is thus in competition with the absorbent core and/or superabsorbent material for the moisture contained in the bodily fluid. The absorbent core and/or superabsorbent material has a strong affinity for the moisture and once the absorbent core and/or superabsorbent material contacts the bodily fluid, the absorbent core and/or superabsorbent material effectively "lock-up" the moisture of the bodily fluid, thereby reducing the amount of moisture available to contact the cyclodextrin complex and release the components of the cyclodextrin complex to provide odor control benefits. The present invention therefore provides a solution to this problem by disposing the cyclodextrin complex in the absorbent article in a layer that is closer to the body-facing surface of the absorbent article than the absorbent core and/or a layer comprising superabsorbent material. This enables the cyclodextrin complex to come in contact with the bodily fluid preferentially before the bodily fluid comes into contact with the absorbent core and/or superabsorbent material. This results in more effective release of the components of the cyclodextrin complex and providing improved odor control benefits.

### CYCLODEXTRIN COMPLEX

The cyclodextrin complex of the present invention comprises cyclodextrin and at least three components complexed with the cyclodextrin.

As used herein, the term "cyclodextrin" includes any of the known cyclodextrins such as substituted and unsubstituted cyclodextrins containing from about six to about twelve glucose units, for example alpha-cyclodextrin, beta-cyclodextrin, gamma-cyclodextrin and/or their derivatives and/or mixtures thereof. For example, the cyclodextrin complex of the present invention can comprise cyclodextrin selected from the group consisting of beta-cyclodextrin, alpha-cyclodextrin, hydroxypropyl alpha-cyclodextrin, hydroxypropyl beta-cyclodextrin, methylated-alpha-cyclodextrin, methylated-beta-cyclodextrin, and mixtures thereof.

The components complexed with the cyclodextrin can be selected from a number of different components. The cyclodextrin complex comprises at least three components that are complexed with cyclodextrin. Suitable components include fragrance components and reactive components. Fragrance components are typically used in the field of perfumery to provide a composition with an aesthetically pleasing scent. Reactive components include components that can react with malodors, such as ammonia-based malodors or sulphur-based malodors (i.e. "malodor reactive components"), and components that mask malodors and/or react with receptors of the nose to block the perception of malodor by the nose of a consumer (i.e. "malodor masking components"). Suitable reactive components are described, for example, in US 2008/0071238 A1 and WO 2007/113778 A2.

In terms of reactive components, those reacting with ammonia or sulphur can be very effective in the cyclodextrin complex of the present invention. Ammonia is one component of malodor associated with the absorption bodily fluids, such as menses or urine. For example, ammonia is typically present in high amounts in absorbent products used for urine absorption due to degradation of urea. Ammonia and its derivatives can react with aldehydes and/or ketones to form imines (according to the so-called Schiff base reaction). 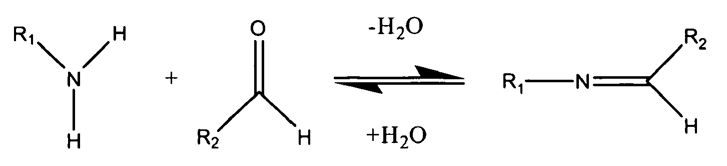 This reaction is catalyzed by enzymes and/or by a slightly acidic pH 4 to 5. The moderate acid requirement is necessary to allow protonation of the hydroxyl intermediate to allow water to leave.

Many aldehydes and ketones capable of imine reaction have an unpleasant and/or too intense odor that can be disturbing to human nose and/or they are very volatile and so not stable in the product. Therefore, selected aldehydes and/or ketones for controlling such malodors are used. Examples of suitable aldehydes and ketones for controlling malodour are those aldehydes and ketones that are able to react with amine compounds according to Schiff base reaction and have not unpleasant odor. Suitable aldehydes include hexyl cinnamic aldehyde, alpha-amylcinnamic aldehyde, p-anisaldehyde, 4-Formyl-2-methoxyphenyl 2-methylpropanoate, benzaldehyde, cinnamic aldehyde, cuminic aldehyde, decanal, p-t-butyl-alpha-methyldihydrocinnamaldehyde, 4-hydroxy-3-methoxycinnamaldehyde, 2-phenyl-3-(2-furyl)prop-2-enal, vanillin isobutyrate, ethyl vanillin acetate, vanillin acetate, cyclamen aldehyde, heptanal, lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, S-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)-,2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial or mixtures thereof.

Suitable aldehydes can also be selected from hexyl cinnamic aldehyde, decanal, 4-formyl-2-methoxyphenyl 2-methylpropanoate, 4-hydroxy-3-methoxycinnamaldehyde, 3,5-dimethoxy-4-hydroxycinnamaldehyde, 2-phenyl-3-(2-furyl)prop-2-enal, ethyl vanillin acetate, vanillin isobutyrate, vanillin acetate, asaronaldehyde, or mixtures thereof.

Suitable aldehydes can also be selected from hexyl cinnamic aldehyde, 4-hydroxy-3-methoxycinnamaldehyde, decanal, or mixtures thereof.

Suitable ketones include 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, laevo-carvone, or mixtures thereof.

Preferably, the malodor reactive component is selected from the group consisting of hexyl cinnamic aldehyde, alpha-amylcinnamic aldehyde, p-anisaldehyde, benzaldehyde, cinnamic aldehyde, cuminic aldehyde, decanal, cyclamen aldehyde, p-t-butyl-alpha-methyldihydrocinnamaldehyde, 4-hydroxy-3-methoxycinnamaldehyde, vanillin isobutyrate, 2-phenyl-3-(2-furyl)prop-2-enal, ethyl vanillin acetate, vanillin acetate, heptanal, lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)- ,2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial, Laevo-Carvone, 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, and mixtures thereof.

Other components suitable herein are components that mask the malodors or react with receptors of the nose. The components that mask the malodor tend to be volatile materials that modify the vapor pressure of the malodour, thereby reducing the impression of the malodour. The components that mask the malodor can also do so by inhibiting the receptors of the nose. When used, these materials may significantly reduce the capability for the nose to detect the malodors. The nose blocking is possible due to the volatile nature of the materials selected, which are released from the cyclodextrin complex of the absorbent article and are then inhaled into the nose of a consumer, generally within somewhat close range of the absorbent article, e.g. within about 0 to 10 meters of the article by normal breathing (although this should in no way be intended to limit the scope of the invention). The blocking of the nose receptors is, of course, only temporary. Suitable malodor masking components include menthol, menthyl acetate, menthyl lactate, 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, isomenthyl acetate, isomenthyl propionate, isomenthyl isobutyrate, isomenthyl propionate, isomenthyl butyrate, camphor, p-menthane, limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and/or triethyl acetate, para-cresol, para-cymene, methyl abietate, methyl dihydro jasmonate, hexyl-2-methyl butyrate, benzyl acetate, laevo carvone , hexyl-2-methyl butyrate , eucalyptus, phenyl ethyl alcohol and mixtures thereof. The materials also include their isomeric forms, diastereomers and enantiomers. Advantageously, in general, the above materials have only a very slight inherent odour but show a high degree of malodour masking and/or nose receptor blocking.

Preferably, the malodor masking component is selected from the group consisting of menthol, menthyl acetate, menthyl lactate, 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, isomenthyl acetate, isomenthyl propionate, isomenthyl isobutyrate, isomenthyl propionate, isomenthyl butyrate, camphor, p-menthane, limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and/or triethyl acetate, para-cresol, para-cymene, methyl abietate, methyl dihydro jasmonate, hexyl-2-methyl butyrate, benzyl acetate, laevo carvone , hexyl-2-methyl butyrate , eucalyptus, phenyl ethyl alcohol, and mixtures thereof.

The components complexed with cyclodextrin can also include fragrance components that impart an aesthetically pleasing odor character to the mixture. Suitable fragrance components which can be used in the cyclodextrin complex include limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, di-hydrocumarin, di-hydromyrcenyl acetate, geraniol, geranial, encalyptus, isoamylacetate, ethyl, and /or triethyl acetate, para-cresol and para-cymene, benzyl-benzoate, isopropyl myristate, methyl abietate, ethanol, isopropanol, diethylene glycol monoethyl ether, glycerol, propylene glycol, 1,2-butylene glycol, dipropylene glycol, 2-methyl-2,4-pentanediol, diethyl phthalate, triethyl citrate, diethyl sebacate.

It may be that, for certain components, the same component can be considered both a malodor reactive component, a malodor masking component, and/or a fragrance component. This is fine so long as the cyclodextrin complex contains at least three different specific components.

In the context of an absorbent article, it is preferred that the absorbent article exhibits no noticeable scent (or very little scent) before use. As a result, it is preferred that the cyclodextrin complex has a low amount of free components that are not complexed with the cyclodextrin. In accordance with at least some of the preferred embodiments, the percent of components that are complexed with cyclodextrin is greater than about 75%, greater than about 90%, or greater than about 95%. It should be understood that these levels of component complexation are directly associated with the complex formation process itself; i.e. the percentages do not represent a formulation design of adding a first percentage of components via a cyclodextrin complex and adding a second percentage of neat components.

Cyclodextrin complexes can be formed by various methods which are well known in the art. For example, US 5,543,157 from The Procter & Gamble Company describes methods of forming cyclodextrin complexes.

As one example of a method of forming a cyclodextrin complex, a solvent (e.g., water or an organic solvent suitable for the organic compound to be complexed), unloaded cyclodextrin particles, and the organic compound which need to be complexed can be placed into a container and then mixed for a period of time to permit loading of organic molecules into "cavities" of cyclodextrin molecules. The mixture may or may not be processed further; e.g., processed through a colloid mill and/or homogenizer. The solvent is then substantially removed from the resulting mixture or slurry to yield cyclodextrin complex particles, e.g. via spray drying. Different manufacturing techniques may however impart different particle/complex characterizations, which may or may not be desirable in the absorbent articles, depending on the specific usage and conditions. In some embodiments the particles of cyclodextrin inclusion complexes have a low level of moisture prior to their inclusion into the polysiloxane carrier, typically of less than about 20% by weight of the particles, or of less than about 10% by weight of the particles, or of less than about 6% by weight of the particles. Spray drying a slurry of inclusion complexes of cyclodextrin and organic compounds is one manufacturing technique capable of producing the cyclodextrin particles and cyclodextrin complexes having the above-noted, moisture levels. Cyclodextrin complexes can also be obtained using known techniques and an extrusion process (kneading) however the resulting material will in general contain a higher humidity and a lower complexation efficiency. US 2008/0213191 A1 from The Procter & Gamble Company provides a detailed overview of preferred techniques for preparing cyclodextrin complexes.

The cyclodextrin complex can be applied in a variety of ways, and in a variety of patterns, to the absorbent article. For example, when the cyclodextrin complex is dispersed in a carrier, the dispersion containing the cyclodextrin complex can be applied using conventional glue application equipment such as a slot applicator, which can be used for striped patterns, or air assisted applicators for patterned applications (like spray, spiral, serpentine, fibrils, omega®, signature® and the like) because this allow to position the odour control material in a way that it does not impact fluid acquisition (i.e. in a fem care article the material is not applied in correspondence with the vaginal opening) and the pattern, having a large void space, allows fluid penetration also on the sides. Also patterned applications are helpful because it allows a precise application so that it is easier to avoid contact with the glue which connects the various layers of the absorbent article.

The cyclodextrin complex can applied in powder form or can be incorporated into a carrier and applied as a lotion. The cyclodextrin complex can be dispersed in a carrier to form a dispersion, and the dispersion applied to the absorbent article. The carrier can be selected from the group consisting of polysiloxane oil, mineral oil, petrolatum, polyethylene glycol, glyercin, and the like, and mixtures thereof. The carrier is preferably polysiloxane oil, such as a silicone glycol copolymer (commercially available from Dow Coming as Dow Coming 190 Fluid).

The cyclodextrin complex is typically disposed in the absorbent article in an amount of from about 10 to about 5000 milligrams per absorbent article, from about 20 to about 1000 milligrams per absorbent article, from about 30 to about 500 milligrams per absorbent article, or from about 70 to about 300 milligrams per absorbent article.

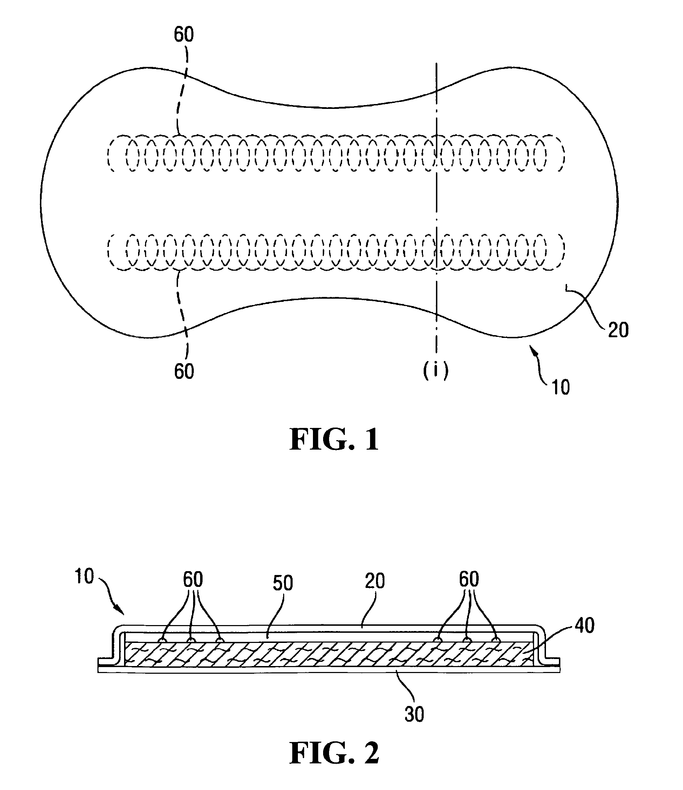

FIG. 1 shows an absorbent article, such as a sanitary napkin, according to the present invention. FIG. 2 is a cross-sectional view of the same absorbent article along the line indicated by (i) in FIG. 1. The absorbent article (10) comprises a topsheet (20), a backsheet (30), an absorbent core (40), a secondary topsheet (50) and two spirals of cyclodextrin complex (60) according to the present invention applied to the body-facing surface of the absorbent core (40). The cyclodextrin complex (60) is therefore disposed in a layer of the absorbent article (10) that is closer to the body-facing surface of the absorbent article (10) than the absorbent core (40).

The present invention further encompasses a method of reducing malodor associated with bodily fluid such as urine, menses, and/or feces, comprising the step of contacting the bodily fluid with an absorbent article of the present invention. Preferably, the method reduces the malodor associated with menses.

### HEADSPACE TEST METHOD

The following headspace test method measures the amount of components complexed with cyclodextrin that are released into the headspace surrounding a sample of an absorbent article before and after insult with Artificial Menstrual Fluid ("AMF").

For this test method, the Artificial Menstrual Fluid used is based on modified sheep's blood that has been modified to closely resemble human menstrual fluid in viscosity, electrical conductivity, surface tension and appearance. It is prepared as explained in US Patent 6,417,424, assigned to The Procter & Gamble Company, from line 33 of column 17 to line 45 of column 18, to which reference is made.

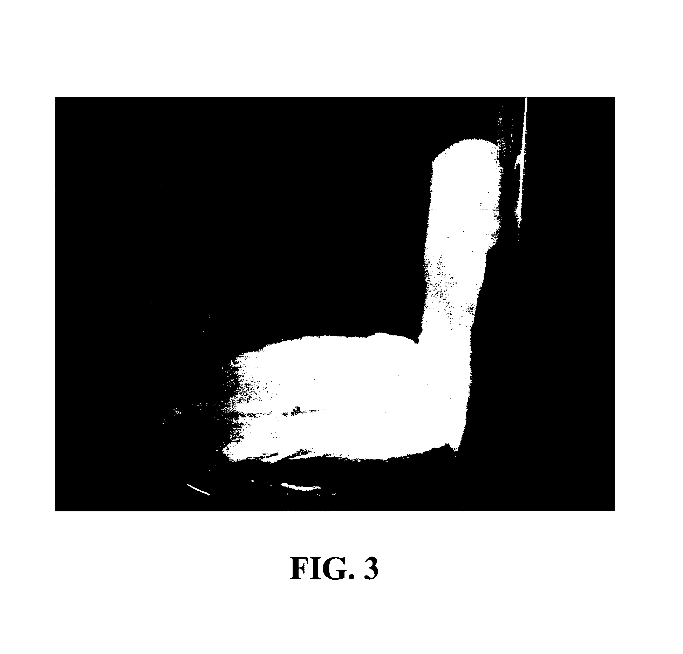

At the bottom of a 1700 ml rounded glass vial (11 cm in diameter x 19 cm in height and having a metal cover that seals the glass vial), the absorbent article to be tested is placed such that the central portion of the absorbent article is positioned on the bottom of the glass vial (with the topsheet of the absorbent article facing up). In the case of a sanitary napkin, if the sanitary napkin has wings (or side flaps), the wings are first folded under the sanitary napkin (adjacent the backsheet of the sanitary napkin) and the end portions of the sanitary napkin are folded up in order to fit the sanitary napkin completely in the glass vial and to allow the central portion of the sanitary napkin to sit on the bottom of the glass vial. The positioning of the sanitary napkin in the glass vial is illustrated in FIG. 3. If a larger absorbent article is to be tested, then the ends and/or sides of the absorbent article can be cut shorter, in order to just fit the absorbent article entirely in the glass vial and to allow the central portion of the absorbent article to sit on the bottom of the glass vial.

Once the absorbent article to be tested is loaded into the glass vial, the glass vial is sealed with a metal cover. The metal cover has a circular hole, 1 mm - 2 mm in diameter, in the center of the cover. The circular hole in the metal cover is sealed with a piece of office tape (e.g. SCOTCH MAGIC tape from 3M) adhered on the top surface of the cover, covering and sealing the circular hole.

Immediately after the glass vial containing the absorbent article is sealed with the metal cover, a headspace sample is taken. The headspace sample is taken with a SPME Fiber Assembly Polydimethylsiloxane (a SPME fiber coated with 100µm PDMS, needle size 24 gauge, for use with manual holder, available from Supelco as Model Number 57300-U). The headspace sample is taken with the SPME Fiber Assembly by piercing the office tape covering the circular hole in the metal cover for the glass vial and exposing the fiber to the headspace in the glass vial according to the instructions provided with the SPME Fiber Assembly PDMS (Model Number 57300-U from Supelco) for 15 minutes. Immediately after taking the sample, the circular hole in the metal cover of the glass vial is re-sealed with another piece of office tape. The SPME fiber is then desorbed to a gas chromatography-mass spectrometry ("GC-MS") instrument, as follows, for 2 minutes:

| | |
|---|---|
| GC-MS Instrument: | Polaris Q from Thermo Fisher Scientific |
| Software: | Xcalibur Version 1.3 |
| Column Used: | VF-5ms L-30m, ID=0.25mm, Ft=1.0µm |

### Gas Chromatography Conditions:

| | |
|---|---|
| Temperature: | 90°C (2min) → 7°C/min → 260°C (6min) |
| Injection: | PTV splitless 260°C - splitless time 0.8 |
| Carrier: | Constant flow 1.5ml/min |
| MS Transfer Line: | 250°C |

### Mass Spectrometry Conditions:

| | |
|---|---|
| EI positive | |
| Ion source: | 250°C |

The GC-MS instrument generates a chromatogram with peaks corresponding to each component in the headspace sample. The operator of the instrument identifies those peaks which correspond to the components originally complexed with cyclodextrin of the cyclodextrin complex of the absorbent article (for this, the operator has to know the components originally complexed with the cyclodextrin; if the operator needs to first determine the components originally complexed with the cyclodextrin, a sample of the absorbent article containing the cyclodextrin complex can be tested using a modified Headspace Test Method described herein in which the absorbent article sample is placed in a 1700 ml glass vial, then insulted with 10 ml of water, then the glass vial is sealed and placed in a laboratory oven for 4 hours at 37°C, then the headspace is sampled using the SPME fiber described herein, and then the headspace sample is analyzed with the GC-MS instrument as described herein to identify the components in the headspace which will correspond to the components originally complexed with the cyclodextrin). The areas under the peaks corresponding to those components originally complexed with cyclodextrin are determined and added together. The sum of these areas is then reported as the "Total Headspace Area at Time 0 Minutes" for the absorbent article sample.

The metal cover of the glass vial is then removed and 10 ml of AMF are added with a calibrated micropipette (e.g. FINNPIPETTE available from Sigma-Aldrich) to the central portion of the absorbent article sample in the glass vial in an area of about 3cm x 8cm. The glass vial is then immediately re-sealed with the metal cover. The glass vial is then placed in a laboratory oven at 37°C. After 30 minutes, another headspace sample is taken from the glass vial (according to the procedure described above for the measurement at time 0 minutes). The headspace sample is taken directly in the laboratory oven for 15 minutes and then desorbed to the GC-MS instrument for 2 minutes. The sum of the areas on the chromatogram that correspond to the components originally complexed with the cyclodextrin are reported as the "Total Headspace Area" at time 30 minutes for the absorbent article sample.

The glass vial containing the absorbent article sample then remains in the laboratory oven at 37°C and headspace samples are then taken at time 80 minutes, 120 minutes, and 240 minutes. The sum of the areas on the chromatogram that correspond to the components originally complexed with the cyclodextrin are reported as the "Total Headspace Area" at time 80 minutes, 120 minutes, and 240 minutes, respectively.

The test method is repeated two more times on additional samples of the same type of absorbent article. The average (mean) of the three Total Headspace Area values at each time interval are reported. The standard deviation of the average Total Headspace Area value is approximately 10% at each time interval.

### EXAMPLE 1

This is an example of an absorbent article of the present invention wherein the cyclodextrin complex is disposed on the garment-facing side of the secondary topsheet of the absorbent article.

The cyclodextrin complex is prepared as follows. The following components are added in order in a mildly agitated vessel, to create movement at the top of fluid, but without creating air bubbles: 55 grams of distilled water, 41 grams of beta cyclodextrin (contains nominally 12% moisture), and 4 grams of the Component Mixutre of Table 1 below.

| **TABLE 1: COMPONENT MIXTURE** | |
|---|---|
| **INGREDIENT** | **AMOUNT (wt%)** |
| Menthyl Acetate | 20.000 |
| Xandralia 992420¹ | 20.000 |
| Methyl Dihydro Jasmonate | 30.000 |
| Benzyl Acetate | 7.000 |
| Tetra Hydro Linalool | 9.000 |
| Laevo Carvone | 0.200 |
| Hexyl-2-methyl Butyrate | 2.000 |
| Eucalyptus | 0.300 |
| Linalyl Acetate | 3.500 |
| Phenyl Ethyl Alcohol | 8.000 |

| | |
|---|---|
| ¹ available from Firmenich | |

The resulting slurry is agitated for 30 minutes and then passed through a colloid mill (Gaulin mill). The rheology of the solution changes to a viscous slurry as the complexation occurs. The slurry is then dried via nozzle spray drying at an inlet temperature of approximately 195°C and an outlet temperature of about 98°C. The resulting cyclodextrin complex is a powder having a moisture content of about 5%, by weight of the cyclodextrin complex, and a content of components complexed with cyclodextrin of about 8% to about 9%, by weight of the cyclodextrin complex. The cyclodextrin complex has less than about 2% of components that are uncomplexed with the cyclodextrin.

A LINES PETALO BLU CON ALI sanitary napkin, commercially available from Fater SpA, Italy, is obtained. The release paper wrapper of the sanitary napkin is removed and the sanitary napkin is unfolded into a flat, unfolded configuration. The sanitary napkin is then cut along one longitudinal side of the article (leaving the other longitudinal side intact). The topsheet is separated from the secondary topsheet ("STS"). On the garment-facing side of the STS, 50 milligrams of the cyclodextrin complex is applied in the center of the STS in an area of 3 cm x 8 cm (a spatula is used to apply the cyclodextrin complex uniformly). The sanitary napkin is re-assembled in its original order and orientation, and a new thermal seal is provided along the cut longitudinal side.

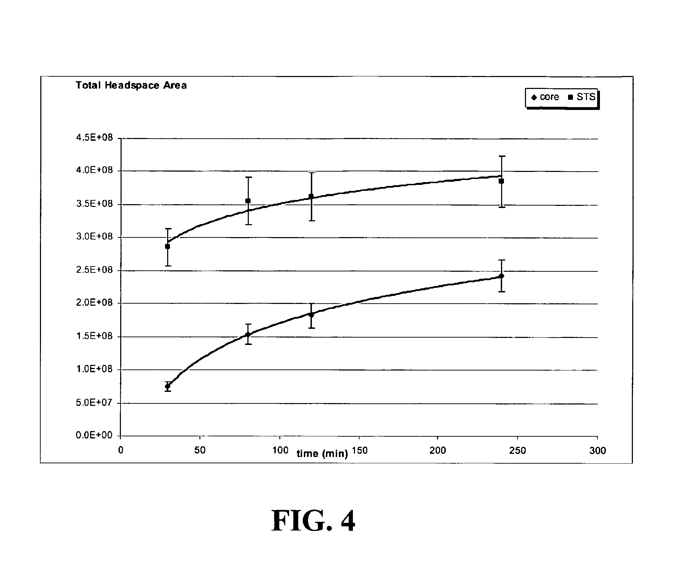

The resulting sanitary napkin is subjected to the Headspace Test Method described herein and the Total Headspace Area values are plotted in the graph of FIG. 4.

### COMPARATIVE EXAMPLE

This is a comparative example of an absorbent article wherein the cyclodextrin complex is disposed in the absorbent core of the absorbent article.

A cyclodextrin complex is prepared as in Example 1.

A LINES PETALO BLU CON ALI sanitary napkin, commercially available from Fater SpA, Italy, is obtained. The release paper wrapper of the sanitary napkin is removed and the sanitary napkin is unfolded into a flat, unfolded configuration. The sanitary napkin is then cut along one longitudinal side of the article (leaving the other longitudinal side intact). The secondary topsheet ("STS") is separated from the absorbent core. The absorbent core is cut in half, thereby forming a top layer and a bottom layer of the absorbent core. On the body-facing side of the bottom layer of the absorbent core, 50 milligrams of the cyclodextrin complex is applied in the center of the bottom layer of the absorbent core in an area of 3 cm x 8 cm (a spatula is used to apply the cyclodextrin complex uniformly). The sanitary napkin is re-assembled in its original order and orientation, and a new thermal seal is provided along the cut longitudinal side.

The resulting sanitary napkin is subjected to the Headspace Test Method described herein and the Total Headspace Area values are plotted in the graph of FIG. 4.

The Total Headspace Area values plotted in the graph of FIG. 4 illustrate that disposing the cyclodextrin complex on the garment-facing side of the STS provides significantly better release of odor control components from the cyclodextrin complex over a period of 240 minutes as compared to disposing the cyclodextrin complex in the absorbent core of the absorbent article.

### EXAMPLE 2

This is an example of an absorbent article of the present invention wherein the cyclodextrin complex is formulated with a carrier and disposed on the garment-facing side of the secondary topsheet of the absorbent article.

A cyclodextrin complex is prepared as described in Example 1. 40 grams of the cyclodextrin complex are added slowly to 60 grams of a silicon glycol copolymer (Dow Coming 190 Fluid) in a mixer while stirring, obtaining a homogeneous dispersion which is kept under stirring.

A sanitary napkin, ALWAYS Ultra Regular available from The Procter & Gamble Company, is cut along a longitudinal side (leaving the other longitudinal side intact). The topsheet is separated from the secondary topsheet ("STS"). On the garment-facing side of the STS, 170 milligrams of the dispersion containing Dow Coming 190 Fluid and the cyclodextrin complex is applied in two thin spirals similar to those shown in Fig. 1. The sanitary napkin is re-assembled in its original order and orientation, and a new thermal seal is provided along the cut longitudinal side.

### EXAMPLE 3

This is an example of an absorbent article of the present invention wherein the cyclodextrin complex is formulated with a carrier and disposed on the garment-facing side of the secondary topsheet of the absorbent article.

A cyclodextrin complex is prepared as described in Example 1, except the Component Mixture has the following formulation as shown in Table 2:

| **TABLE 2: COMPONENT MIXTURE** | |
|---|---|
| **INGREDIENT** | **AMOUNT (wt%)** |
| Chamomille Base 199213¹ | 50 |
| Hexyl Salicylate | 18 |
| Triethyl Citrate | 30 |
| Vanillin Isobutyrate | 1 |
| Vanillin Acetate | 1 |

| | |
|---|---|
| ¹ available from Firmenich | |

40 grams of the cyclodextrin complex are added slowly to 60 grams of a silicon glycol copolymer (Dow Coming 190 Fluid) in a mixer while stirring, obtaining a homogeneous dispersion which is kept under stirring.

A sanitary napkin, ALWAYS Ultra Regular available from The Procter & Gamble Company, is cut along a longitudinal side (leaving the other longitudinal side intact). The topsheet is separated from the secondary topsheet ("STS"). On the garment-facing side of the STS, 170 milligrams of the dispersion containing PDMS and the cyclodextrin complex is applied in two thin spirals similar to those shown in Fig. 1. The sanitary napkin is re-assembled in its original order and orientation, and a new thermal seal is provided along the cut longitudinal side.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An absorbent article having a body-facing surface and a garment-facing surface, said absorbent article comprising:
a topsheet;
a backsheet;
an absorbent core disposed between said topsheet and said backsheet; and
a cyclodextrin complex comprising cyclodextrin and at least three components complexed with said cyclodextrin, wherein said cyclodextrin complex is disposed in a layer of said absorbent article that is closer to said body-facing surface of said absorbent article than said absorbent core.

2. The absorbent article of Claim 1, wherein said at least three components comprise at least one reactive component and at least one fragrance component.

3. The absorbent article of any one of the preceding claims, wherein said reactive component is selected from the group consisting of a malodor reactive component, a malodor masking component, and mixtures thereof.

4. The absorbent article of any one of the preceding claims, wherein said at least three components comprise a malodor reactive component, a malodor masking component, and a fragrance component.

5. The absorbent article of any one of Claims 3-4, wherein said malodor reactive component is selected from the group consisting of hexyl cinnamic aldehyde, alpha-amylcinnamic aldehyde, p-anisaldehyde, benzaldehyde, cinnamic aldehyde, cuminic aldehyde, decanal, cyclamen aldehyde, p-t-butyl-alpha-methyldihydrocinnamaldehyde, 4-hydroxy-3-methoxycinnamaldehyde, vanillin isobutyrate, 2-phenyl-3-(2-furyl)prop-2-enal, ethyl vanillin acetate, vanillin acetate, heptanal, lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)- ,2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial, Laevo-Carvone, 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, and mixtures thereof.

6. The absorbent article of any one of Claims 3-5, wherein said malodor masking component is selected from the group consisting of menthol, menthyl acetate, menthyl lactate, 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-l-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, isomenthyl acetate, isomenthyl propionate, isomenthyl isobutyrate, isomenthyl propionate, isomenthyl butyrate, camphor, p-menthane, limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and/or triethyl acetate, para-cresol, para-cymene, methyl abietate, methyl dihydro jasmonate, hexyl-2-methyl butyrate, benzyl acetate, laevo carvone , hexyl-2-methyl butyrate , eucalyptus, phenyl ethyl alcohol, and mixtures thereof.

7. The absorbent article of any one of the preceding claims, wherein said cyclodextrin is selected from the group consisting of alpha-cyclodextrin and beta-cyclodextrin.

8. The absorbent article of any one of the preceding claims, wherein said cyclodextrin complex is dispersed in a polysiloxane oil to form a dispersion, wherein said dispersion is applied to said absorbent article.

9. The absorbent article of any one of the preceding claims, wherein said absorbent article is a sanitary napkin.

10. The absorbent article of any one of the preceding claims, wherein said absorbent article further comprises a layer of material disposed between said topsheet and said absorbent core.

11. The absorbent article of Claim 10, wherein said cyclodextrin complex is disposed on said layer of material disposed between said topsheet and said absorbent core.

12. The absorbent article of Claim 11, wherein said layer of material disposed between said topsheet and said absorbent core is a secondary topsheet.

13. The absorbent article of Claim 12, wherein said secondary topsheet has a body-facing surface and a garment-facing surface, wherein said cyclodextrin complex is disposed on said garment-facing surface of said secondary topsheet.

14. The absorbent article of any one of the preceding claims, wherein said absorbent core comprises superabsorbent material.

15. A method of reducing malodor associated with menses comprising the step of contacting said menses with an absorbent article of any one of the preceding claims.
